# FASCICULE DE BREVET EUROPEEN

(11) **EP 2 086 603 B1**
(45) Date de publication et mention de la délivrance du brevet: **02.01.2013**
(21) Numéro de dépôt: 07870242.0
(22) Date de dépôt: 05.11.2007
(51) Int. Cl.: A61L 31/10, A61L 29/14, A61L 27/34, A61L 27/54, A61L 27/56, A61L 29/08, A61L 29/16, A61L 31/14, A61L 31/16, A61K 47/40, A61K 47/48, C08B 37/00

(54) **PROCEDE DE PREPARATION D'UNE PROTHESE IMPLANTABLE A PARTIR D'UN CORPS DE BASE EN MATERIAU POREUX, PROTHESE ET UTILISATION ASSOCIEES**
VERFAHREN ZUR HERSTELLUNG EINER IMPLANTIERBAREN PROTHESE AUS EINEM KÖRPER MIT PORÖSER BASIS UND RELEVANTE PROTHESE UND IHRE VERWENDUNG
METHOD FOR PREPARING AN IMPLANTABLE PROSTHESIS FROM A POROUS BASE BODY AND ASSOCIATED PROSTHESIS AND USE THEREOF

(30) Priorité: 06.11.2006 FR 0609678; 26.10.2007 FR 0758629
(43) Date de publication de la demande: 12.08.2009
(73) Titulaire: Laboratoires Perouse, 60173 Ivry Le Temple (FR)
(72) Inventeur: MONDOT, Johanne, 60000 Beauvais (FR); HAULON, Stephan, 59000 Lille (FR); HILDEBRAND, Hartmut Friedrich, 59120 Lille (FR); BLANCHEMAIN, Nicolas Jean Yves, 59660 Merville (FR); MARTEL, Bernard Gérard Emmanuel, 59850 Nieppe (FR)
(74) Mandataire: Jacobson, Claude
(86) Numéro de dépôt international: PCT/FR2007/001822
(87) Numéro de publication internationale: WO 2008/068400

(56) Documents cités:
- WO-A-00/47630
- WO-A-00/47811
- WO-A-2006/051227
- DE-A1- 19 849 464
- US-A1- 2006 058 867
- US-E1- R E38 091

## Description

La présente invention concerne un procédé de préparation d'une prothèse implantable à partir d'un corps de base en matériau poreux, le procédé comprenant les étapes successives suivantes :
a) application sur le corps d'un revêtement de réception d'un agent bioactif, le revêtement de réception comprenant :
   - au moins une cyclodextrine et/ou au moins un dérivé de cyclodextrine et/ou au moins un complexe d'inclusion de cyclodextrine et/ou de dérivés de cyclodextrine,
   - au moins un acide poly(carboxylique),
   - et éventuellement d'un catalyseur ;
b) chauffage du corps à une température comprise entre 100°C et 220°C, pendant une durée de 1 à 90 minutes ;
c) lavage à l'eau du corps ;
d) séchage du corps, puis
e) éventuellement, imprégnation du corps dans une solution concentrée d'au moins un agent bioactif.

Selon un mode de réalisation, le procédé de préparation d'une prothèse implantable selon l'invention comprend l'étape e) d'imprégnation du corps dans une solution concentrée d'au moins un agent bioactif.

Par « revêtement de réception d'un agent bioactif », on entend un revêtement susceptible de recevoir au moins un agent bioactif, mais qui ne reçoit pas nécessairement cet agent bioactif.

On connaît des prothèses tubulaires vasculaires en polyéthylène téréphtalate implantées chez des patients souffrant de problèmes artériels. Ces prothèses ont pour but notamment de remplacer des artères bouchées ou rétractées. Dans ce cas, une dérivation est réalisée autour de l'artère bouchée à l'aide d'une prothèse tubulaire de diamètre variable.

De telles prothèses vasculaires sont également utilisées pour éviter les ruptures d'anévrisme. Une prothèse est alors introduite au sein même de l'artère, en regard de l'anévrisme, afin d'empêcher l'anévrisme d'évoluer.

L'implantation de ces prothèses réduit donc notablement la mortalité chez les patients atteints de ces problèmes artériels.

Toutefois, des complications peuvent apparaître en raison des risques d'infection per-opératoires et post-opératoires respectivement avant et après l'implantation de ces prothèses. Pour pallier ce problème, la demande WO 2006/051227 propose de lier des cyclodextrines sur le matériau constituant un implant destiné à être placé dans le corps humain. L'implant est ensuite placé dans un bain afin de charger les cyclodextrines par des molécules de principes actifs en vue de leur relarguage dans le corps après l'opération.

Un tel procédé est mis en oeuvre au niveau d'un laboratoire. Toutefois, les implants produits par ce procédé ne sont pas satisfaisants en vue de leur implantation finale dans le corps humain, notamment en terme d'étanchéité, d'hémocompatibilité et plus généralement, pour répondre aux contraintes réglementaires liées aux prothèses.

Un but de l'invention est donc d'obtenir un procédé de préparation d'une prothèse implantable qui permette d'obtenir des prothèses diminuant le risque d'infection post-opératoire tout en répondant aux contraintes d'implantation dans le corps humain.

A cet effet, l'invention a pour objet un procédé du type précité, caractérisé en ce que le procédé comprend, après l'étape de séchage, une étape de dépôt d'un revêtement additionnel d'étanchéification sur le corps.

La Demanderesse a par ailleurs mis en évidence que, de manière inattendue, les cyclodextrines liées sur le matériau implantable confèrent une activité bactériostatique, voire bactéricide, audit matériau implantable sans même qu'un principe actif, en particulier tel qu'un antibiotique, antimicrobien, antiseptique ou antiadhésif, ait été chargé sur les cyclodextrines.

Le procédé selon l'invention peut comprendre une ou plusieurs des caractéristiques suivantes, prise(s) isolément ou suivant toutes combinaisons techniquement possibles :
- le revêtement d'étanchéification est formé à base de collagène, de gélatine, de polyuréthane ou de leurs combinaisons ;
- l'étape d'application du revêtement de réception comporte l'imprégnation du corps avec une solution aqueuse comprenant :
   - au moins une cyclodextrine et/ou au moins un dérivé de cyclodextrine et/ou au moins un complexe d'inclusion de cyclodextrine et/ou de dérivés de cyclodextrine,
   - au moins un acide poly(carboxylique),
   - et éventuellement d'un catalyseur ;
- il comprend, avant l'étape de chauffage, une étape de précuisson du corps à une température comprise entre 40°C et 100°C ;
- il comporte, après l'étape de séchage, une étape de neutralisation du corps pour l'amener à un pH neutre ;
- le corps est formé d'une tresse, d'un tissage ou d'un tricot d'au moins un fil polymère, réalisé notamment à base d'un polymère choisi parmi le groupe constitué par le polytéréphtalate d'éthylène (PET), le polyéthylène teréphtalate glycol (PETG), le polyuréthane (PU) l'acide polylactique, l'acide polyglycolique et leurs copolymères, le fluorure de polyvinylidène (PVDF), le polytétrafluoroéthylène (PTFE), le polyéthylène glycol (PEG), le polyamide-6, le polyamide-6,6, le polypropylène, le polyéthylène et leurs copolymères, ou la combinaison de ces polymères;
- le revêtement de réception forme entre 5% et 7% de la masse totale de la prothèse ;
- ledit éventuel agent bioactif est sélectionné parmi les anticoagulants, les anti-thrombogéniques, les agents anti-mitotiques, les agents anti-prolifération, antiadhésion, anti-migration, les promoteurs d'adhésion cellulaire, les facteurs de croissance, les molécules antiparasitaires, les anti-inflammatoires, les angiogéniques, les inhibiteurs de l'angiogenèse, les vitamines, les hormones, les protéines, les antifongiques, les molécules antimicrobiennes, les antiseptiques ou les antibiotiques ; et
- il comprend, avant l'étape d'application du revêtement de réception et/ou après l'étape de lavage, une étape de gaufrage du corps.
- le corps est un corps tubulaire, le corps tubulaire muni desdits revêtements délimitant intérieurement un conduit étanche de circulation d'un liquide.

L'invention a en outre pour objet une prothèse implantable comportant :
- un corps de base en matériau poreux ;
- un revêtement de réception d'un agent bioactif appliqué sur le corps, le revêtement comprenant au moins une cyclodextrine et/ou au moins un dérivé de cyclodextrine et/ou au moins un complexe d'inclusion de cyclodextrine et/ou de dérivés de cyclodextrine,
- éventuellement, au moins un agent bioactif reçu dans le revêtement de réception ;
caractérisé en ce que la prothèse comprend un revêtement additionnel d'étanchéification déposé sur le corps.

Selon un mode de réalisation, la prothèse implantable selon l'invention comprend au moins un agent bioactif reçu dans le revêtement de réception.

L'invention concerne en outre l'utilisation d'au moins une cyclodextrine et/ou au moins un dérivé de cyclodextrine et/ou au moins un complexe d'inclusion de cyclodextrine et/ou de dérivés de cyclodextrine, pour la fabrication d'un dispositif médical bactériostatique.

Selon des variantes,
- l'utilisation définie ci-dessus est pour la fabrication d'un dispositif médical bactéricide,
- le dispositif médical est destiné à entrer en contact avec du plasma sanguin ou tout autre liquide ou tissu biologique,
- le dispositif médical est implantable au moins partiellement dans le corps d'un patient,
- le dispositif est une prothèse implantable qui comporte :
   - un corps de base en matériau poreux ;
   - un revêtement de réception d'un agent bioactif appliqué sur le corps, le revêtement comprenant ladite au moins une cyclodextrine et/ou au moins un dérivé de cyclodextrine et/ou au moins un complexe d'inclusion de cyclodextrine et/ou de dérivés de cyclodextrine,
- éventuellement, au moins un agent bioactif reçu dans le revêtement de réception ;
la prothèse comprenant un revêtement additionnel d'étanchéification déposé sur le corps.

Selon un mode de réalisation, la prothèse comporte :
- un corps de base en matériau poreux ;
- un revêtement de réception d'un agent bioactif appliqué sur le corps, le revêtement comprenant ladite au moins une cyclodextrine et/ou au moins un dérivé de cyclodextrine et/ou au moins un complexe d'inclusion de cyclodextrine et/ou de dérivés de cyclodextrine,
- éventuellement, au moins un agent bioactif reçu dans le revêtement de réception ;
la prothèse comprenant un revêtement additionnel d'étanchéification déposé sur le corps.

Sans vouloir être tenu par un mécanisme d'action, la cyclodextrine et/ou dérivé de cyclodextrine et/ou complexe d'inclusion de cyclodextrine et/ou dérivés de cyclodextrine agiraient au moins en réduisant la capacité d'adhérence des bactéries sur le dispositif médical et, en conséquence, le développement des bactéries. Les cyclodextrines confèrent donc des propriétés bactériostatiques, voire bactéricides, au dispositif médical.

Cet effet bactériostatique ou bactéricide conféré par les cyclodextrines se manifeste notamment lorsque le dispositif médical a été en contact avec du plasma sanguin, par exemple pendant 24 heures. Ainsi, la cyclodextrine et/ou dérivé de cyclodextrine et/ou complexe d'inclusion de cyclodextrine et/ou dérivés de cyclodextrine peuvent être préférentiellement utilisés pour fabriquer un dispositif médical qui est destiné à entrer en contact avec du plasma sanguin ou tout autre liquide ou tissu biologique, par exemple un dispositif qui est implantable au moins partiellement dans le corps d'un patient, ledit dispositif médical exerçant ses propriétés bactériostatiques ou bactéricides lorsqu'il est mis en contact avec le plasma sanguin ou tout autre liquide ou tissu biologique, notamment lorsqu'il est implanté partiellement dans le corps du patient.

Par « bactériostatique » on désigne ici la capacité d'un matériau à inhiber, empêcher, prévenir, la multiplication de populations bactériennes. Un effet bactériostatique peut être médié ou non par la mort des bactéries.

Par « bactéricide » on désigne ici la capacité d'un matériau à réduire ou détruire des populations bactériennes, généralement en tuant les bactéries.

La prothèse selon l'invention peut comprendre l'une ou plusieurs des caractéristiques suivantes, prise(s) isolément ou suivant toutes combinaisons techniquement possibles :
- le corps présente une structure chimique qui comporte une fonction hydroxyle et/ou une fonction amine, ladite structure étant liée de façon covalente à au moins une molécule de cyclodextrine ou à un polymère composé de cyclodextrine du revêtement de réception dont la structure comporte la répétition du motif de formule générale : 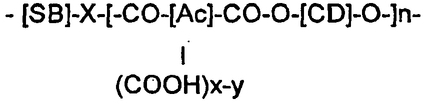 SB représentant la structure chimique du corps constitué d'un matériau polymère d'origine naturelle ou artificielle, X étant soit un atome d'oxygène soit un groupe NH, x > 3, et 2<y <(x-1) et (x-y) >1,
- x étant le nombre de fonctions carboxyle portées par l'acide poly(carboxylique) avant réaction,
- y représente le nombre de fonctions carboxyle de l'acide poly(carboxylique) estérifiées ou amidifiées lors de la réaction,
- (x-y) est le nombre de fonctions carboxyle de l'acide poly(carboxylique) non estérifiées ou amidifiées après réaction, 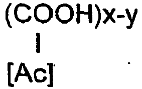 représentant la chaîne moléculaire d'un acide poly(carboxylique) de formule 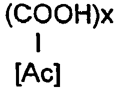 dont au moins deux fonctions carboxyle (-COOH) ont subi une estérification ou subi respectivement une estérification et une amidification et qui porte au moins une fonction carboxyle n'ayant pas subi de réaction d 'estérification ou d'amidification ;
- [CD] représentant la structure moléculaire des cyclodextrines choisies préférentiellement parmi : l'α-cyclodextrine, la β-cyclodextrine, la γ-cyclodextrine et leurs mélanges ; leurs dérivés qui sont choisis préférentiellement parmi les dérivés aminés, méthylés, hydroxypropylés, sulfobutylés, carboxyméthylés, ou carboxyliques, et leurs mélanges, ledit agent bioactif étant au moins une molécule thérapeutique formant un complexe avec les molécules de cyclodextrine et/ou de dérivés de cyclodextrine ;
- le revêtement de réception est formé par un polymère réticulé d'au moins une cyclodextrine et/ou d'au moins un dérivé de cyclodextrine et/ou d'au moins un complexe d'inclusion de cyclodextrine ou de dérivés de cyclodextrine et dont la structure comporte la répétition d'un motif de formule générale : 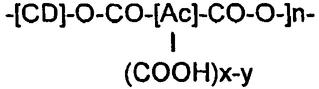 avec x > 3, et 2<y ≤(x-1) et (x-y)≥1,
- x étant le nombre de fonctions carboxyle portées par l'acide poly(carboxylique) avant réaction,
- y représentant le nombre de fonctions carboxyle de l'acide poly(carboxylique) estérifiées lors de la réaction,
- (x-y) étant le nombre de fonctions carboxyle de l'acide poly(carboxylique) non estérifiées après réaction 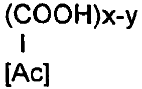 représentant la chaîne moléculaire d'un acide poly(carboxylique) de formule: 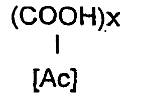 dont au moins deux fonctions carboxyle (-COOH) ont subi une estérification et qui porte au moins une fonction carboxyle n'ayant pas subi de réaction d'estérification;
- [CD] représentant la structure moléculaire des cyclodextrines choisies préférentiellement parmi: l'α-cyclodextrine, la β-cyclodextrine, la γ-cyclodextrine et leurs mélanges; leurs dérivés qui sont choisis préférentiellement parmi les dérivés aminés, méthylés, hydroxypropylés, sulfobutylés, carboxyméthylés, ou carboxyliques, et leurs mélanges, ledit agent bioactif étant une molécule thérapeutique formant un complexe avec les molécules de cyclodextrine et/ou de dérivés de cyclodextrine ;
- le matériau poreux est formé d'une tresse, d'un tissage, ou d'un tricot d'au moins un fil polymère, notamment réalisé à base d'un polymère choisi parmi le groupe constitué par le polytéréphtalate d'éthylène (PET), le polyéthylène teréphtalate glycol (PETG), le polyuréthane (PU) l'acide polylactique, l'acide polyglycolique et leurs copolymères, le fluorure de polyvinylidène (PVDF), le polytétrafluoroéthylène (PTFE), le polyéthylène glycol (PEG), le polyamide-6, le polyamide-6,6, le polypropylène, le polyéthylène et leurs copolymères, ou la combinaison de ces polymères ;
- le revêtement de réception forme entre 5% et 7% de la masse totale de la prothèse ;
- l'éventuel agent bioactif est choisi parmi les anticoagulants, les anti-thrombogéniques, les agents anti-mitotiques, les agents anti-prolifération, anti-adhésion, antimigration, les promoteurs d'adhésion cellulaire, les facteurs de croissance, les molécules antiparasitaires, anti-inflammatoires, les antifongiques, les molécules antimicrobiennes, les antiseptiques et les antibiotiques ;
- le corps est gaufré ; et
- le revêtement d'étanchéité est formé à base de collagène, de gélatine, de polyuréthane
   ou de leurs combinaisons.
- le corps est un corps tubulaire, le corps tubulaire muni desdits revêtements délimitant intérieurement un conduit étanche de circulation d'un liquide.

L'invention sera mieux comprise à la lecture de la description qui va suivre, donnée uniquement à titre d'exemple, et faite en se référant aux dessins annexés, sur lesquels :
- la Figure 1 est une vue en perspective de côté d'une première prothèse tubulaire selon l'invention ;
- la Figure 2 est une vue schématique des premières étapes d'un procédé de fabrication d'une prothèse tubulaire selon l'invention ;
- la Figure 3 est une vue analogue à la Figure 2 des étapes du procédé de fabrication postérieures aux étapes représentées sur la Figure 2 ; et
- la Figure 4 est une vue analogue à la Figure 1 d'une deuxième prothèse selon l'invention.

L'invention s'applique notamment aux prothèses, aux endoprothèses vasculaires ou aux stents présentant un corps tubulaire droit ou bifurqué.

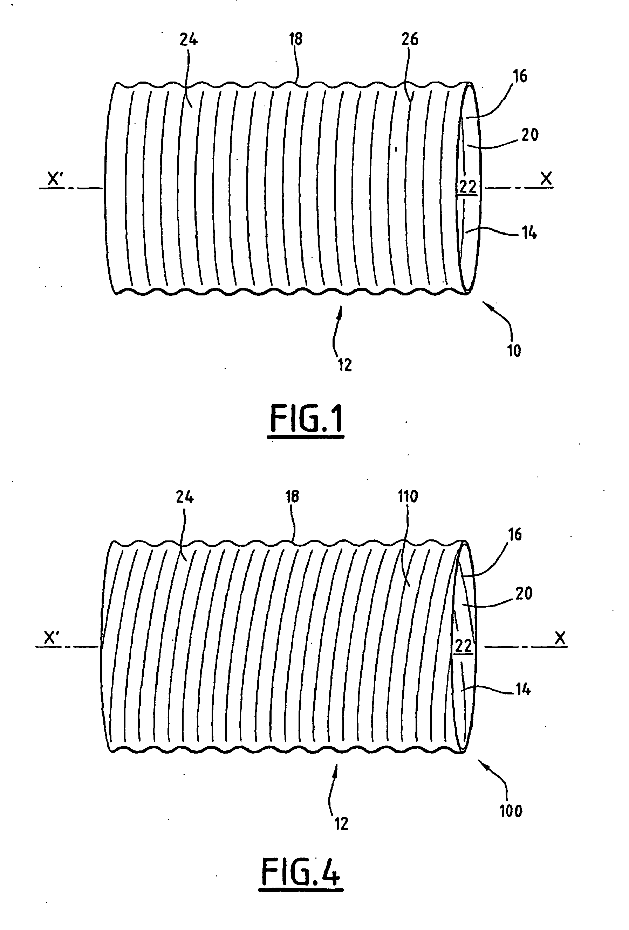

Une première prothèse 10 selon l'invention est représentée sur la Figure 1. Comme illustré par cette Figure, cette prothèse comprend un corps tubulaire 12 gaufré présentant extérieurement un revêtement 14 de réception d'un agent bioactif et un revêtement 16 d'étanchéification de la prothèse.

Le corps tubulaire 12 comprend une paroi tubulaire 18 sensiblement cylindrique d'axe X-X' présentant une surface intérieure 20 délimitant un conduit 22 de circulation du sang et une surface extérieure 24 destinée à être appliquée au moins partiellement contre un tissu humain.

La paroi tubulaire 18 est réalisée à base de fils de polymère entrelacés, sous forme d'une tresse, d'un tissage, ou d'un tricot.

Le ou chaque fil polymère est réalisé à base d'un polymère choisi parmi le groupe constitué par le polytéréphtalate d'éthylène (PET), le polyéthylène teréphtalate glycol (PETG), le polyuréthane (PU) l'acide polylactique, l'acide polyglycolique et leurs copolymères, le fluorure de polyvinylidène (PVDF), le polytétrafluoroéthylène (PTFE), le polyéthylène glycol (PEG), le polyamide-6, le polyamide-6,6, le polypropylène, le polyéthylène et leurs copolymères, ou la combinaison de ces polymères.

Le ou chaque fil polymère délimite à travers la paroi 18 une pluralité de pores s'étendant entre la surface intérieure 20 et la surface extérieure 24. La paroi tubulaire 18 est donc poreuse en l'absence des revêtements 14, 16.

Dans l'exemple représenté sur la Figure 1, la paroi 18 est gaufrée ou « cosselée ». Elle présente ainsi une pluralité de nervures annulaires 26 faisant saillie vers l'extérieur.

Les revêtements 14, 16 sont appliqués sur les fils constituant la paroi 18 et dans les pores délimités entre ces fils, suivant la surface intérieure 20 et la surface extérieure 24. Les revêtements 14, 16 coopèrent pour obturer ainsi sensiblement la totalité des pores, de sorte que le corps tubulaire 12 muni des revêtements 14, 16, délimite intérieurement un conduit étanche 22 de circulation du sang d'axe X-X'.

Le revêtement 14 de réception d'un agent bioactif comprend au moins une cyclodextrine et/ou un dérivé de cyclodextrine et/ou un complexe d'inclusion de cyclodextrine et/ou de dérivé de cyclodextrine. Il comprend en outre un acide poly(carboxylique) et un catalyseur. Le revêtement 14 est lié à la paroi tubulaire 18 suivant l'un au moins des deux modes de liaison décrits ci-dessous.

Dans un premier mode de liaison, la paroi tubulaire 18 présente une structure chimique qui comporte une fonction hydroxyle et/ou une fonction amine, ladite structure étant liée de façon covalente à au moins une molécule de cyclodextrine ou à un polymère composé de cyclodextrine du revêtement de réception dont la structure comporte la répétition du motif de formule générale : 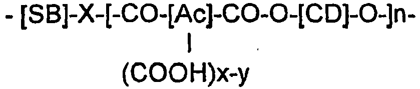 SB représentant la structure chimique du corps tubulaire constitué d'un matériau polymère d'origine naturelle ou artificielle, X étant soit un atome d'oxygène soit un groupe NH, x > 3, et 2<y <(x-1) et (x-y) >1,
- x étant le nombre de fonctions carboxyle portées par l'acide poly(carboxylique) avant réaction,
- y représente le nombre de fonctions carboxyle de l'acide poly(carboxylique) estérifiées ou amidifiées lors de la réaction,
- (x-y) est le nombre de fonctions carboxyle de l'acide poly(carboxylique) non estérifiées ou amidifiées après réaction, 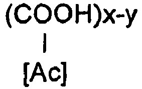 représentant la chaîne moléculaire d'un acide poly(carboxylique) de formule 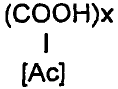 dont au moins deux fonctions carboxyle (-COOH) ont subi une estérification ou subi respectivement une estérification et une amidification et qui porte au moins une fonction carboxyle n'ayant pas subi de réaction d'estérification ou d'amidification ;
- [CD] représentant la structure moléculaire des cyclodextrines choisies préférentiellement parmi : l'α-cyclodextrine, la β-cyclodextrine, la γ-cyclodextrine et leurs mélanges ;

Dans ce cas, le revêtement est obtenu par la formation d'un anhydre de l'acide poly(carboxylique) qui réagit avec le matériau constituant la paroi tubulaire 18 en formant une liaison covalente de type amide ou ester entre ce matériau et l'acide poly(carboxylique). Ensuite, dans le cas de figure le plus simple, un second anhydre de l'acide poly(carboxylique) déjà lié au matériau est formé, celui-ci réagit avec une molécule de cyclodextrine ou une molécule de cyclodextrin ancrée en une liaison ester avec la molécule de cyclodextrine ou de dérivé de cyclodextrine.

Dans un deuxième mode de liaison, le revêtement de réception est formé par un polymètre réticulé d'au moins une cyclodextrine et/ou d'au moins un dérivé de cyclodextrine et/ou d'au moins un complexe d'inclusion de cyclodextrine ou de dérivés de cyclodextrine et dont la structure comporte la répétition d'un motif de formule générale : 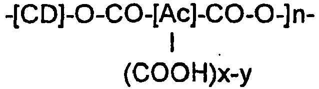 avec x > 3, et 2<y ≤(x-1) et (x-y)≥1,
- x étant le nombre de fonctions carboxyle portées par l'acide poly(carboxylique) avant réaction,
- y représentant le nombre de fonctions carboxyle de l'acide poly(carboxylique) estérifiées lors de la réaction,
- (x-y) étant le nombre de fonctions carboxyle de l'acide poly(carboxylique) non estérifiées après réaction 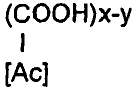 représentant la chaîne moléculaire d'un acide poly(carboxylique) de formule: 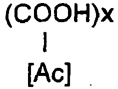 dont au moins deux fonctions carboxyle (-COOH) ont subi une estérification et qui porte au moins une fonction carboxyle n'ayant pas subi de réaction d'estérification ;
- [CD] représentant la structure moléculaire des cyclodextrines choisies préférentiellement parmi: l'α-cyclodextrine, la β-cyclodextrine, la γ-cyclodextrine et leurs mélanges.

Dans ce deuxième mode, la fixation de la ou de chaque cyclodextrine et/ou de dérivés de cyclodextrine est réalisée par la formation d'un polymètre réticulé obtenu par la réaction exclusive entre les molécules de cyclodextrine et/ou de dérivés de cyclodextrine et au moins un acide poly(carboxylique). Le polymère réticulé ainsi formé forme un film ou un dépôt en surface des fils de la paroi tubulaire 18 ou est consigné à l'intérieur de sa structure poreuse et y reste fixé de façon permanente.

En variante, les deux modes de liaison coexistent sur les fils constituant la paroi 18.

Dans cet exemple, le revêtement d'étanchéification 16 est formé par un revêtement de collagène déposé sur les fils formant la paroi tubulaire 18, dans les pores délimités entre les fils, et sur le revêtement de réception 14. Le collagène forme un film obturant les interstices entre la surface intérieure 20 et la surface extérieure 24 pour former le conduit étanche 22 à l'intérieur du corps tubulaire 12.

En variante, le revêtement d'étanchéification 16 est réalisé à base de gélatine, de polyuréthane ou de tout autre polymère susceptible d'étanchéifier le corps tubulaire 12, ou de leur mélange.

L'éventuel agent bioactif est reçu dans la structure moléculaire creuse constituée par chaque cyclodextrine. Il est sélectionné parmi les anticoagulants, les anti-thrombogéniques, les agents anti-mitotiques, les agents anti-prolifération, antiadhésion, anti-migration, les promoteurs d'adhésion cellulaire, les facteurs de croissance, les molécules antiparasitaires, les anti-inflammatoires, les angiogéniques, les inhibiteurs de l'angiogenèse, les vitamines, les hormones, les protéines, les antifongiques, les molécules antimicrobiennes, les antiseptiques ou les antibiotiques.

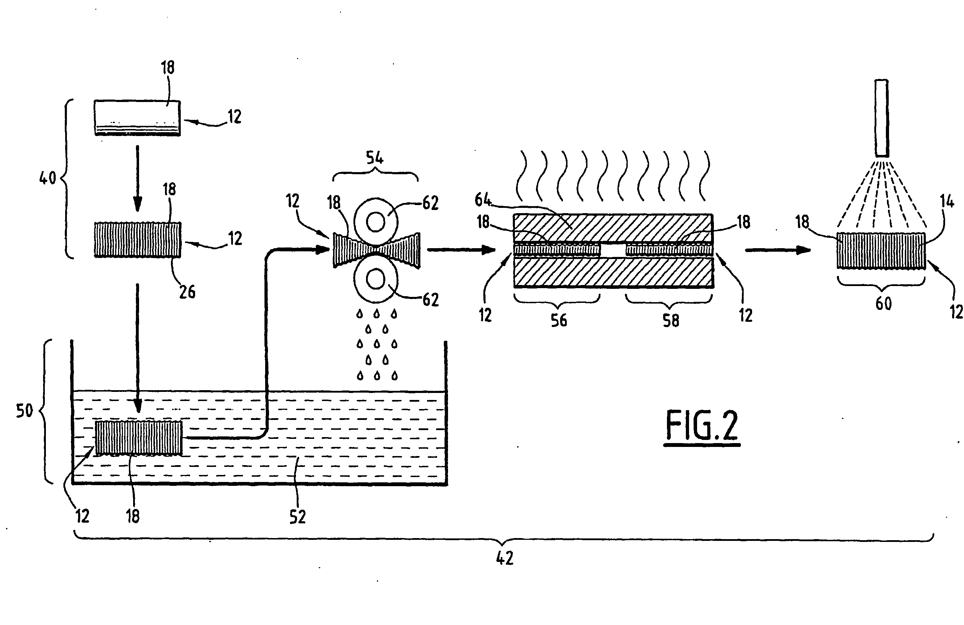

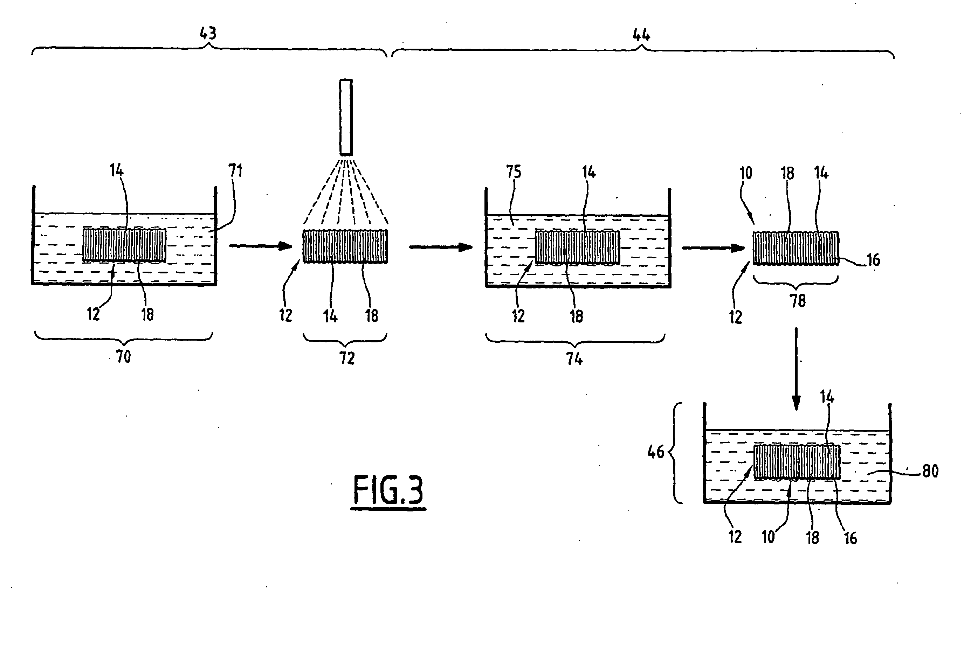

Le procédé de préparation de la prothèse 10 selon l'invention va maintenant être décrit, en regard des Figures 2 et 3.

Ce procédé comprend une étape 40 de mise en forme du corps tubulaire 12, une étape 42 d'application du revêtement de réception 14, une étape 43 de neutralisation, une étape d'application 44 du revêtement d'étanchéification 16, puis, éventuellement, une étape 46 de chargement de l'agent bioactif sur la prothèse 10.

Initialement, à l'étape 40 de mise en forme, un corps tubulaire 12 tressé, tissé ou tricoté à base de fils polymères est fourni. Ce corps tubulaire 12 présente initialement une surface extérieure 24 et une surface intérieure 20 lisses et une paroi tubulaire 18 poreuse.

Un gaufrage de la paroi tubulaire 18 constituant le corps est ensuite réalisé pour former les nervures 26.

L'étape 42 d'application du revêtement 14 comprend l'imprégnation 50 dans une solution 52 de mélange précurseur de chaque corps 12 mis en forme, le foulardage 54 de chaque corps 12 imprégné de mélange précurseur, une précuisson 56, une cuisson 58 et un lavage et séchage 60 de chaque corps 12.

Le mélange précurseur 52 est formé par une solution aqueuse comportant :
- au moins une cyclodextrine et/ou au moins un dérivé de cydodextrine et/ou au moins un complexe d'inclusion de cyclodextrine et/ou dé dérivés de cyclodextrine,
- au moins un acide poly(carboxylique),
- et éventuellement d'un catalyseur.

La cyclodextrine est choisie de préférence parmi l'α-cyclodextrine, la β-cyclodextrine la γ-cyclodextrine et leurs mélanges ; leurs dérivés qui sont choisis préférentiellement parmi les dérivés aminés, méthylés, hydroxypropylés, sulfobutylés, carboxy méthylés, ou carboxyliques de ces cyclodextrines, et leurs mélanges.

L'acide carboxylique est choisi parmi les acides poly(carboxytiques) acycliques saturés et insaturés cycliques saturés et insaturés aromatiques, les acides hydroxypoly(carboxyliques), de préférence, l'acide citrique, l'acide polyacrylique, l'acide poly(méthacrylique), l'acide 1, 2, 3, 4-butanetétracarboxylique, l'acide maléique, l'acide citraconique, l'acide itaconique, l'acide 1, 2, 3-propanetricarboxylique, l'acide trans-aconitique, l'acide all-cis-1, 2, 3, 4-cyclopentanetétracarboxylique, l'acide méllittique, l'acide oxydisuccinique, l'acide thiodisuccinique, ou parmi les anhydrides dérivés des acides sus nommés.

Le catalyseur est choisi parmi les dihydrogénophosphates, les hydrogénophosphates, les phosphates, les hypophosphites, les phosphites de métaux alcalins, les sels de métaux alcalins des acides polyphosphoriques, les carbonates, les bicarbonates, les acétates, les borates, les hydroxydes de métaux alcalins, les aminés aliphatiques et l'ammoniaque, et de préférence, parmi l'hydrogénophosphate de sodium, le dihydrogénophosphate de sodium et phypophosphite de sodium.

De préférence, le mélange est constitué à partir d'acide poly(carboxylique), de catalyseur et de cyclodextrine dans les proportions suivantes en masse : acide poly(carboxylique) : entre 2 et 4, catalyseur : 1, cyclodextrine : entre 2 et 5.

Le foulardage 54 est réalisé par passage des corps tubulaires 12 imprégnés de mélange précurseur entre deux rouleaux 62, et en enserrant le corps tubulaire 12 entre les rouleaux 62.

La précuisson 56 est réalisée de préférence dans un four 64 porté à une température comprise entre 40°C et 100°C pendant une période de temps comprise entre 1 minutes et 30 minutes. Les surfaces 20 et 24 du corps 12 sont alors sensiblement sèches.

L'étape de chauffage est réalisée dans le four 64 à une température comprise entre 100°C et 220°C pendant une durée de 1 à 90 minutes.

Le taux de retenue du revêtement 14 de réception ainsi formé est compris entre 5% et 7% par rapport à la masse totale de la prothèse 10 après séchage. Ce taux préserve les propriétés physiques et mécaniques de la prothèse 10, comme par exemple la souplesse, la piquabilité et la flexibilité.

A l'étape de neutralisation 43, chaque corps tubulaire 12 est plongé dans une solution aqueuse de carbonate d'alcalin, par exemple du carbonate de sodium.

La concentration de cette solution est comprise entre 1 g/l et 5 g/l. Le temps d'immersion de chaque corps tubulaire 12 dans la solution de neutralisation est par exemple compris entre 1 minutes et 60 minutes.

Puis, un lavage 72 au soxhlet à l'eau est effectué sur trois cycles, suivi d'un séchage complet des prothèses 10 munies uniquement du revêtement 14 de réception. Le pH de la surface intérieure et de la surface extérieure est alors sensiblement égal à 7.

L'étape 44 d'application du revêtement de collagène comprend l'immersion 74 du corps tubulaire 12 dans une solution aqueuse de collagène 76. Le collagène est par exemple choisi parmi les collagènes de type IV issus d'une peau de veau. La concentration de collagène dans la solution est comprise entre 2% et 4% en masse. Le taux de retenue de collagène sur le corps 12 est par exemple compris entre 20% et 30% en masse de la masse totale du corps 12 muni des revêtements 14, 16 formant la prothèse 10 après séchage.

Le collagène est par exemple fourni à base d'une solution aqueuse à pH acide fournie par la société française SYMATHESE. Dans la solution de collagène, on ajoute du glycérol pour améliorer la souplesse, et on ajoute de l'eau pour amener la solution de collagène à pH neutre, c'est-à-dire environ à un pH de 7.

Le séchage 78 de la prothèse 10 est ensuite effectué. Ensuite, la prothèse 10 est stérilisée à l'aide d'un rayonnement ionisant β.

Lorsqu'elle est mise en oeuvre, l'étape 46 de chargement du principe actif sur la prothèse 10 est par exemple réalisée par le chirurgien juste avant l'opération. A cet effet, la prothèse 10 est plongée dans un bain 80 contenant l'agent bioactif. Cet agent bioactif est sélectionné parmi les anticoagulants, les anti-thrombogéniques, les agents anti-mitotiques, les agents anti-prolifération, antiadhésion, anti-migration, les promoteurs d'adhésion cellulaire, les facteurs de croissance, les molécules antiparasitaires, les anti-inffammatoires, les angiogéniques, les inhibiteurs de l'angiogenèse, les vitamines, les hormones, les protéines, les antifongiques, les molécules antimicrobiennes, les antiseptiques ou les antibiotiques.

Le procédé qui vient d'être décrit permet donc d'obtenir des prothèses qui peuvent être avantageusement chargées d'un agent bioactif qui sera libéré au cours du temps dans l'organisme après son implantation dans le corps humain.

La coopération entre un revêtement 14 de réception du principe actif et un revêtement 16 d'étanchéification permet d'obtenir des prothèses qui présentent une étanchéité très satisfaisante en vue de leur implantation dans le corps humain. La coopération locale entre les revêtements 14, 16 diminue la quantité de collagène utilisée tout en assurant une bonne étanchéité, ce qui limite les risques liés à la présence du collagène d'origine animale.

De manière surprenante, la présence du revêtement d'étanchéification 16 n'affecte pas la capacité du revêtement de réception 14 à se charger en agent bioactif, ni sa capacité à diffuser cet agent une fois implanté dans le corps humain.

Les quantités de revêtement de réception 14 et de revêtement d'étanchéification 16 déposées sur le corps tubulaire 12 ont été optimisées pour assurer de bonnes propriétés physiques et mécaniques (souplesse, piquabilité, imperméabilité, flexibilité...)

En outre, la neutralisation de la surface intérieure 20 et de la surface extérieure 24 de la prothèse 10 assure l'hémocompatibilité de la paroi 18 revêtues des revêtements 14, 16 en vue de l'implantation ultérieure de la prothèse dans le corps humain et de la circulation du sang au sein de la prothèse.

La présence de gaufrages sur la prothèse 10 améliore en outre l'accroche du revêtement de réception 14 sur la surface intérieure 20 et sur la surface extérieure 24 de la prothèse 14, ce qui diminue la concentration des solutions 52 de mélange précurseur et évite les problèmes de plicature.

Une deuxième prothèse 100 selon l'invention est représentée sur la Figure 4. A la différence de la prothèse 10, elle présente une rainure hélicoïdale unique 110.

La surface intérieure 20 forme donc une première surface isolée de manière étanche de la deuxième surface formée par la surface extérieure 24, lorsque le corps 12 est muni des revêtements 14, 16.

Dans une autre variante, le corps 12 est formé par un assemblage de fibres polymères non tissées, liées entre elles par exemple par un adhésif.

Dans une autre variante, la prothèse est un patch vasculaire comprenant un corps 12 qui n'est pas tubulaire. Le corps 12 est alors sensiblement plan et présente une épaisseur nominale de paroi inférieure à 0,6 mm et une résistance à la suture transversale supérieure ou égale à 15 N. Le corps 12 délimite ainsi une surface supérieure sensiblement plane et une surface inférieure sensiblement plane isolée de manière étanche par les revêtements 14, 16.

Un autre avantage de l'invention tient à la nature du revêtement susceptible de recevoir l'agent bioactif, ledit revêtement comprenant une cyclodextrine et/ou un dérivé de cyclodextrine et/ou un complexe d'inclusion de cyclodextrine.

En effet, la Demanderesse a démontré qu'une prothèse telle que définie plus haut comportant un revêtement à base de cyclodextrine et/ou dérivé de cyclodextrine et/ou complexe d'inclusion de cyclodextrine, présente une activité bactériostatique sans toutefois que ce revêtement n'ait été imprégné d'un agent bioactif.

Cette activité bactériostatique propre à la cyclodextrine, ou à ses dérivés ou complexes d'inclusion, a été mise en évidence dans un test d'adhérence bactérienne effectué contre un témoin constitué d'une prothèse dépourvue de revêtement à base de cyclodextrine et/ou dérivé de cyclodextrine et/ou complexe d'inclusion de cyclodextrine.

La mise en évidence de ces propriétés est détaillée dans l'exemple ci-après, dans lesquelles les prothèses ont été mises en contact avec du plasma sanguin.

Ces résultats démontrent l'intérêt plus général d'utiliser au moins une cyclodextrine et/ou dérivé de cyclodextrine et/ou complexe d'inclusion de cyclodextrine pour la fabrication d'un dispositif médical bactériostatique, voire bactéricide, ce dispositif étant notamment une prothèse ou un biomatériau implantable.

Un tel dispositif est par exemple une prothèse vasculaire ou un patch vasculaire, un fil de suture, un cathéter de dialyse, de perfusion, de nutrition artificielle, une chambre à cathéter implantable, une valve cardiaque, un stent coronaire, un stent périphérique nu ou couvert, une endoprothèse thoracique, un drain, un implant dentaire, un renfort de paroi (hemie/éventration), un implant transcutané, une membrane de régénération tissulaire guidé, une membrane de régénération osseuse guidée, un dispositif de libération intrasucculaire, un treillis et réseau pour ingénierie tissulaire, un substitut osseux micro et macroporeux, un pansement à usage médical et vétérinaire.

Exemple : Mise en évidence d'une activité bactériostatique propre au revêtement de cyclodextrine et/ou dérivé de cyclodextrine et/ou complexe d'inclusion de cyclodextrine.

L'impact du revêtement de cyclodextrine sur la capacité d'adhérence de bactéries sur une prothèse tubulaire conforme à l'invention a été évalué.

Pour cela, un test d'adhérence bactérienne a été mis en oeuvre sur deux types de prothèses vasculaires :
- une prothèse vasculaire « cycloD PMC », constituée d'un corps tubulaire en polyester tissé ou tricoté, revêtu de collagène (15 % p/p) et de HpBCyclodextrines (hydroxypropyle-β-cyclodextrines, 15% p/p) ;
- une prothèse vasculaire « PMC », constituée d'un corps tubulaire en polyester tissé ou tricoté, revêtu de collagène (30 % p/p).

Des disques de 1 cm de diamètre ont été préparés à partir des prothèses vasculaires cycloD PMC ou PMC.

Quatre disques de chacune des prothèses cycloD PMC et PMC ont été immergés séparément dans 12 ml de plasma à 37°C pendant 24 heures, sous agitation.

Ensuite, l'ensemble des disques a été rincé dans un tampon DPBS (tampon phosphate salin de Dulbecco).

Deux disques par prothèse ont ensuite été immergés pendant 1 heure dans une suspension de bactérienne (10⁴ UFC/ml) de *Staphyloccocus aureus* (ATCC 6538) ou *Pseudomonas aerugenosa* (ATCC 9027), puis rincés avec un tampon DPBS (deux disques par échantillon de prothèse pour chaque souche bactérienne).

Les bactéries ont ensuite été récoltées à partir des disques selon le protocole suivant :
- vibrations ultra-sonores à une fréquence de 47 KHz ± 6 % pendant cinq minutes à température ambiante (+15-25°C) ;
- agitation mécanique à raison de 800 secousses/minute pendant 20 minutes à température ambiante (+15-25°C) ;
- agitation au Vortex pendant 30 secondes à température ambiante (+15-25°C).

Les extraits ont ensuite été dilués, de 10⁻¹ à 10⁻³, et filtrés sur des unités de filtration de 0,45 µm. Les membranes récoltées ont été incubées sur des boîtes de Tryptone soja Agar à une température comprise entre 30 et 35°C, en condition aérobie, pendant 24 heures.

Le nombre de colonies a été déterminé visuellement.

Les résultats obtenus sont montrés dans le tableau 1 ci-dessous.

**Tableau 1 : nombre de microorganismes récoltés pour des disques préalablement incubés 24 heures en plasma :**

| | PMC | | | | CycloD PMC | | | |
|---|---|---|---|---|---|---|---|---|
| | UFC/membrane | | | UFC/disque | UFC/membrane | | | UFC/disque |
| | 10⁻¹ | 10⁻² | 10⁻³ | | 10⁻¹ | 10⁻² | 10⁻³ | |
| *Staphyloccocus aureus* | ND | ND | 224 | 7,5.10⁵ | 287 | 36 | 1 | 9,6.10³ |
| *Pseudomonoas aeruginosa* | ND | ND | 372 | 1,2.10⁶ | 370 | 37 | 3 | 1,2.10⁴ |

| | | | | | | | | |
|---|---|---|---|---|---|---|---|---|
| ND : non déterminé | | | | | | | | |

Après une incubation de 24 heures en plasma, les prothèses PMC étaient recouvertes d'une couche du type feutre blanc qui a été retirée.

Après 24 heures d'immersion dans le plasma, une adhérence bactérienne supérieure de 2 log a été observée pour les prothèses vasculaires PMC par rapport aux prothèses CycloD PMC, démontrant ainsi que le revêtement de cyclodextrine réduit la capacité d'adhérence des bactéries.

Ces résultats indiquent que des prothèses tubulaires implantables qui comportent (i) un corps tubulaire 12 de base en matériau poreux, (ii) un revêtement 14 comprenant au moins une cyclodextrine et/lou au moins un dérivé de cyclodextrine et/ou au moins un complexe d'inclusion de cyclodextrine et/ou de dérivés de cyclodextrine, et (iii) un revêtement additionnel 16 d'étanchéification, constituent en soi des prothèses tubulaires implantables bactériostatiques, voire bactéricides. Des essais sont en cours pour mettre en évidence un effet bactéricide lié au revêtement de cyclodextrine.

De telles prothèses sont donc particulièrement avantageuses puisqu'elles permettent de réduire les risques d'infection post-opératoires même en l'absence d'imprégnation du revêtement de cyclodextrine avec un agent bioactif antibiotique ou antiadhésif, par exemple.

## Revendications

1. Procédé de préparation d'une prothèse (10) implantable à partir d'un corps (12) de base en matériau poreux, le procédé comprenant les étapes successives suivantes :
a) application (50) sur le corps (12) d'un revêtement (14) de réception d'un agent bioactif, le revêtement de réception (14) comprenant :
- au moins une cyclodextrine et/ou au moins un dérivé de cyclodextrine et/ou au moins un complexe d'inclusion de cyclodextrine et/ou de dérivés de cyclodextrine,
- au moins un acide poly(carboxylique),
- et éventuellement d'un catalyseur ;
b) chauffage (58) du corps (12) à une température comprise entre 100°C et 220°C, pendant une durée de 1 à 90 minutes ;
c) lavage (60) à l'eau du corps (12) :
d) séchage (60) du corps (12), puis
e) éventuellement, imprégnation (46) du corps (12) dans une solution concentrée d'au moins un agent bioactif :
**caractérisé en ce que** le procédé comprend, après l'étape de séchage, une étape (44) de dépôt sur le corps (12) d'un revêtement additionnel (16) d'étanchéification à base de collagène, de gélatine, de polyuréthane ou de leurs combinaisons.

2. Procédé selon la revendication 1, **caractérisé en ce que** l'étape (50) d'application du revêtement de réception (14) comporte l'imprégnation du corps avec une solution aqueuse (52) comprenant :
- au moins une cyclodextrine et/ou au moins un dérivé de cyclodextrine et/ou au moins un complexe d'inclusion de cyclodextrine et/ou de dérivés de cyclodextrine,
- au moins un acide poly(carboxylique),
- et éventuellement d'un catalyseur.

3. Procédé selon l'une quelconque des revendications 1 à 2, **caractérisé en ce qu'**il comprend, avant l'étape de chauffage (58), une étape (56) de précuisson (12) du corps à une température comprise entre 40°C et 100°C.

4. Procédé selon l'une quelconque des revendications précédentes, **caractérisé en ce qu'**il comporte, après l'étape de séchage (60), une étape (43) de neutralisation du corps (12) pour l'amener à un pH neutre.

5. Procédé selon l'une quelconque des revendications précédentes, **caractérisé en ce que** le corps (12) est formé d'un assemblage, d'une tresse, d'un tissage ou d'un tricot d'au moins un fil polymère, réalisé notamment à base d'un polymère choisi parmi le groupe constitué par le polytéréphtalate d'éthylène (PET), le polyéthylène teréphtalate glycol (PETG), le polyuréthane (PU) l'acide polylactique, l'acide polyglycolique et leurs copolymères, le fluorure de polyvinylidène (PVDF), le polytétrafluoroéthylène (PTFE), le polyéthylène glycol (PEG), le polyamide-6, le polyamide-6,6, le polypropylène, le polyéthylène et leurs copolymères, ou la combinaison de ces polymères.

6. Procédé selon l'une quelconque des revendications précédentes, **caractérisé en ce que** le revêtement de réception (14) forme entre 5% et 7% de la masse totale de la prothèse (10).

7. Procédé selon l'une quelconque des revendications précédentes **caractérisé en ce qu'**il comprend l'étape e) d'imprégnation (46) du corps (12) dans une solution concentrée d'au moins un agent bioactif.

8. Procédé selon la revendication 7, **caractérisé en ce que** ledit agent bioactif est sélectionné parmi les anticoagulants, les anti-thrombogéniques, les agents anti-mitotiques, les agents anti-prolifération, antiadhésion, anti-migration, les promoteurs d'adhésion cellulaire, les facteurs de croissance, les molécules antiparasitaires, les anti-inflammatoires, les angiogéniques, les inhibiteurs de l'angiogenèse, les vitamines, les hormones, les protéines, les antifongiques, les molécules antimicrobiennes, les antiseptiques ou les antibiotiques.

9. Procédé selon l'une quelconque des revendications précédentes, **caractérisé en ce qu'**il comprend, avant l'étape (50) d'application du revêtement de réception (14) et/ou après l'étape de lavage, une étape (40) de gaufrage du corps (12).

10. Procédé selon l'une quelconque des revendications précédentes, **caractérisé en ce que** le corps (12) est un corps tubulaire, le corps tubulaire (12) muni desdits revêtements (14, 16) délimitant intérieurement un conduit (22) étanche de circulation d'un liquide.

11. Procédé selon l'une quelconque des revendications précédentes, **caractérisé en ce que** le revêtement de réception (14) comprend un dérivé hydroxypropylé de β-cyclodextrine.

12. Prothèse implantable (10) comportant :
- un corps (12) de base en matériau poreux ;
- un revêtement (14) de réception d'un agent bioactif appliqué sur le corps (12), le revêtement comprenant au moins une cyclodextrine et/ou au moins un dérivé de cyclodextrine et/ou au moins un complexe d'inclusion de cyclodextrine et/ou de dérivés de cyclodextrine,
- éventuellement, au moins un agent bioactif reçu dans le revêtement de réception (14) ;
**caractérisé en ce que** la prothèse (10) comprend un revêtement additionnel d'étanchéification (16) à base de collagène, de gélatine, de polyuréthane ou de leurs combinaisons déposé sur le corps (12).

13. Prothèse (10) selon la revendication 12, **caractérisée en ce que** le corps (12) présente une structure chimique qui comporte une fonction hydroxyle et/ou une fonction amine, ladite structure étant liée de façon covalente à au moins une molécule de cyclodextrine ou à un polymère composé de cyclodextrine du revêtement de réception dont la structure comporte la répétition du motif de formule générale : 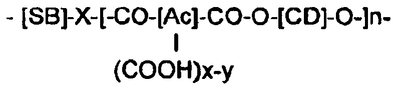 SB représentant la structure chimique du corps constitué d'un matériau polymère d'origine naturelle ou artificielle, X étant soit un atome d'oxygène soit un groupe NH, x > 3, et 2<y <(x-1) et (x-y) >1,
- x étant le nombre de fonctions carboxyle portées par l'acide poly(carboxylique) avant réaction,
- y représente le nombre de fonctions carboxyle de l'acide poly(carboxylique) estérifiées ou amidifiées lors de la réaction,
- (x-y) est le nombre de fonctions carboxyle de l'acide poly(carboxylique) non estérifiées ou amidifiées après réaction, 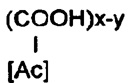 représentant la chaîne moléculaire d'un acide poly(carboxylique) de formule 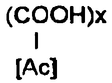 dont au moins deux fonctions carboxyle (-COOH) ont subi une estérification ou subi respectivement une estérification et une amidification et qui porte au moins une fonction carboxyle n'ayant pas subi de réaction d 'estérification ou d'amidification;
- [CD] représentant la structure moléculaire des cyclodextrines choisies préférentiellement parmi : l'α-cyclodextrine, la β-cyclodextrine, la γ-cyclodextrine et leurs mélanges ; leurs dérivés qui sont choisis préférentiellement parmi les dérivés aminés, méthylés, hydroxypropylés, sulfobutylés, carboxyméthylés, ou carboxyliques, et leurs mélanges, ledit agent bioactif étant au moins une molécule thérapeutique formant un complexe avec les molécules de cyclodextrine et/ou de dérivés de cyclodextrine.

14. Prothèse (10) selon l'une des revendications 12 ou 13, **caractérisée en ce que** le revêtement de réception (14) est formé par un polymère réticulé d'au moins une cyclodextrine et/ou d'au moins un dérivé de cyclodextrine et/ou d'au moins un complexe d'inclusion de cyclodextrine ou de dérivés de cyclodextrine et dont la structure comporte la répétition d'un motif de formule générale : 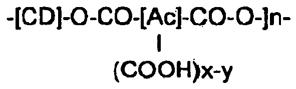 avec x > 3, et 2<y ≤(x-1) et (x-y)≥1,
- x étant le nombre de fonctions carboxyle portées par l'acide poly(carboxylique) avant réaction,
- y représentant le nombre de fonctions carboxyle de l'acide poly(carboxylique) estérifiées lors de la réaction,
- (x-y) étant le nombre de fonctions carboxyle de l'acide poly(carboxylique) non estérifiées après réaction 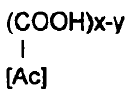 représentant la chaîne moléculaire d'un acide poly(carboxylique) de formule: 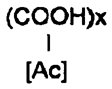 dont au moins deux fonctions carboxyle (-COOH) ont subi une estérification et qui porte au moins une fonction carboxyle n'ayant pas subi de réaction d'estérifcation;
- [CD] représentant la structure moléculaire des cyclodextrines choisies préférentiellement parmi: l'α-cyclodextrine, la β-cyclodextrine, la γ-cyclodextrine et leurs mélanges; leurs dérivés qui sont choisis préférentiellement parmi les dérivés aminés, méthylés, hydroxypropylés, sulfobutylés, carboxyméthylés, ou carboxyliques, et leurs mélanges, ledit agent bioactif étant une molécule thérapeutique formant un complexe avec les molécules de cyclodextrine et/ou de dérivés de cyclodextrine.

15. Prothèse (10) selon l'une quelconque des revendications 12 à 14, **caractérisée en ce que** le matériau poreux est formé d'un assemblage, d'une tresse, d'un tissage, ou d'un tricot d'au moins un fil polymère, notamment réalisé à base d'un polymère choisi parmi le groupe constitué par le polytéréphtalate d'éthylène (PET), le polyéthylène teréphtalate glycol (PETG), le polyuréthane (PU) l'acide polylactique, l'acide polyglycolique et leurs copolymères, le fluorure de polyvinylidène (PVDF), le polytétrafluoroéthylène (PTFE), le polyéthylène glycol (PEG), le polyamide-6, le polyamide-6,6, le polypropylène, le polyéthylène et leurs copolymères, ou la combinaison de ces polymères.

16. Prothèse (10) selon l'une quelconque des revendications 12 à 15, **caractérisée en ce que** le revêtement de réception (14) forme entre 5% et 7% de la masse totale de la prothèse (10).

17. Prothèse (10) selon l'une quelconque des revendications 12 à 16, **caractérisée en ce qu'**elle comporte au moins un agent bioactif reçu dans le revêtement de réception (14).

18. Prothèse (10) selon l'une quelconque des revendications 12 à 17, **caractérisée en ce que** l'agent bioactif est choisi parmi les anticoagulants, les anti-thrombogéniques, les agents anti-mitotiques, les agents anti-prolifération, anti-adhésion, antimigrafion, les promoteurs d'adhésion cellulaire, les facteurs de croissance, les molécules antiparasitaires, anti-inflamatoires, les antifongiques, les molécules antimicrobiennes, les antiseptiques et les antibiotiques.

19. Prothèse (10) selon l'une quelconque des revendications 12 à 18 **caractérisée en ce que** le corps (12) est un corps tubulaire, le corps tubulaire (12) muni desdits revêtements (14, 16) délimitant intérieurement un conduit étanche (22) de circulation d'un liquide.

20. Prothèse (10) selon l'une quelconque des revendications 12 à 19, **caractérisé en ce que** le revêtement (14) de réception comprend un dérivé hydroxypropylé de la β-cyclodextrine.

21. Utilisation d'au moins une cyclodextrine et/ou au moins un dérivé de cyclodextrine et/ou au moins un complexe d'inclusion de cyclodextrine et/ou de dérivés de cyclodextrine, pour la fabrication d'une prothèse (10) implantable bactériostatique susceptible d'être obtenue par le procédé selon l'une quelconque des revendications 1 à 11.

22. Utilisation selon la revendication 21, pour la fabrication d'une prothèse (10) implantable bactéricide.

23. Utilisation selon l'une des revendications 21 ou 22, **caractérisée en ce que** la prothèse (10) implantable est destinée à entrer en contact avec du plasma sanguin ou tout autre liquide ou tissu biologique.

24. Utilisation selon l'une quelconque des revendications 21 à 23, **caractérisée en ce que** le dérivé de cyclodextrine est un dérivé hydroxypropylé de la β-cyclodextrine.

## Claims

1. Method for preparing an implantable prosthesis (10) from a base element (12) made of porous material, the method comprising the following successive steps:
a) applying (50) a coating (14) for receiving a bioactive agent to the element (12), the receiving coating (14) comprising:
- at least one cyclodextrin and/or at least one cyclodextrin derivative and/or at least one cyclodextrin inclusion complex and/or cyclodextrin derivative inclusion complex,
- at least one poly(carboxylic) acid,
- and an optional catalyst;
b) heating (58) the element (12) to a temperature of between 100 °C and 220 °C for between 1 and 90 minutes;
c) washing (60) the element (12) in water;
d) drying (60) the element (12), then
e) optionally impregnating (46) the element (12) in a concentrated solution of at least one bioactive agent;
**characterised in that** the method comprises a step (44) of depositing an additional sealing coating (16) formed on the basis of collagen, gelatin, polyurethane or combinations thereof on the element (12) after the drying step.

2. Method according to claim 1, **characterised in that** the step (50) of applying the receiving coating (14) comprises impregnating the element with an aqueous solution (52) comprising:
- at least one cyclodextrin and/or at least one cyclodextrin derivative and/or at least one cyclodextrin inclusion complex and/or cyclodextrin derivative inclusion complex,
- at least one poly(carboxylic) acid,
- and an optional catalyst.

3. Method according to any one of claims 1 to 2, **characterised in that** it comprises a step (56) of pre-curing (12) the element at a temperature of between 40 °C and 100 °C before the heating step (58).

4. Method according to any one of the preceding claims, **characterised in that** it comprises a step (43) of neutralising the element (12) to bring it to a neutral pH after the drying step (60).

5. Method according to any one of the preceding claims, **characterised in that** the element (12) is formed from an assembly, a mesh, a woven material or a knitted material formed from at least one polymer thread, formed in particular on the basis of a polymer selected from the group consisting of polyethylene terephthalate (PET), polyethylene terephthalate glycol (PETG), polyurethane (PU), polylactic acid, polyglycolic acid and the copolymers thereof, polyvinylidene fluoride (PVDF), polytetrafluoroethylene (PTFE), polyethylene glycol (PEG), polyamide 6, polyamide 6,6, polypropylene, polyethylene and the copolymers thereof, or a combination of these polymers.

6. Method according to any one of the preceding claims, **characterised in that** the receiving coating (14) forms between 5 % and 7 % of the total weight of the prosthesis (10).

7. Method according to any one of the preceding claims, **characterised in that** it comprises the step e) of impregnating (46) the element (12) in a concentrated solution of at least one bioactive agent.

8. Method according to claim 7, **characterised in that** said bioactive agent is selected from anticoagulants, antithrombotics, anti-mitotic agents, anti-proliferation agents, anti-adhesive agents, anti-migration agents, cell adhesion promoters, growth factors, anti-parasitic molecules, anti-inflammatories, pro-angiogenic agents, angiogenesis inhibitors, vitamins, hormones, proteins, anti-fungal agents, anti-microbial molecules, antiseptics or antibiotics.

9. Method according to any one of the preceding claims, **characterised in that** it comprises a step (40) of embossing the element (12) before the step (50) of applying the receiving coating (14) and/or after the washing step.

10. Method according to any one of the preceding claims, **characterised in that** the element (12) is a tubular element, the tubular element (12) provided with said coatings (14, 16) internally delimiting a sealed liquid circulation channel (22).

11. Method according to any one of the preceding claims, **characterised in that** the receiving coating (14) comprises a hydroxypropylated derivative of a β-cyclodextrin.

12. Implantable prosthesis (10) comprising:
- a base element (12) made of porous material,
- a coating (14) for receiving a bioactive agent applied to the element (12), the coating comprising at least one cyclodextrin and/or at least one cyclodextrin derivative and/or at least one cyclodextrin inclusion complex and/or cyclodextrin derivative inclusion complex,
- optionally, at least one bioactive agent received in the receiving coating (14);
**characterised in that** the prosthesis (10) comprises an additional sealing coating (16) formed on the basis of collagen, gelatin, polyurethane or combinations thereof deposited on the element (12).

13. Prosthesis (10) according to claim 12, **characterised in that** the element (12) has a chemical structure which comprises a hydroxyl function and/or an amine function, said structure being covalently bonded to at least one cyclodextrin molecule or to a polymer composed of cyclodextrin of the receiving coating, the structure of which comprises the repetition of the pattern of the general formula: 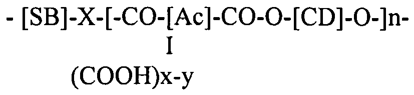 SB representing the chemical structure of the element formed from a polymer material of natural or artificial origin, X being either an oxygen atom or an NH group, x > 3, and 2<y <(x-1) and (x-y) >1,
- x being the number of carboxyl functions carried by the poly(carboxylic) acid before the reaction,
- y representing the number of carboxyl functions of the poly(carboxylic) acid which are esterified or amidified during the reaction,
- (x-y) being the number of carboxyl functions of the poly(carboxylic) acid which are not esterified or amidifed after the reaction, 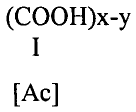 representing the molecular chain of a poly(carboxylic) acid of the formula 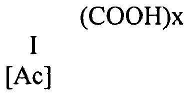
at least two carboxyl functions (-COOH) of which have undergone esterification or esterification and amidification respectively and which carries at least one carboxyl function which has not undergone an esterification or amidification reaction;
- [CD] representing the molecular structure of the cyclodextrins, preferably selected from: α-cyclodextrin, β-cyclodextrin, γ-cyclodextrin and mixtures thereof, the derivatives thereof, preferably selected from the aminated, methylated, hydroxypropylated, sulphobutylated, carboxymethylated or carboxylic derivatives thereof, and mixtures thereof, said bioactive agent being at least one therapeutic molecule which forms a complex with the cyclodextrin molecules and/or cyclodextrin derivatives.

14. Prosthesis (10) according to either claim 12 or claim 13, **characterised in that** the receiving coating (14) is formed by a cross-linked polymer of at least one cyclodextrin and/or of at least one cyclodextrin derivative and/or of at least one cyclodextrin inclusion complex or cyclodextrin derivative inclusion complex, the structure of which comprises the repetition of a pattern of the general formula: 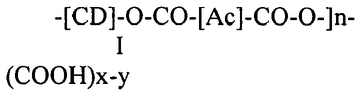 where x > 3, and 2<y ≤(x-1) and (x-y)≥1,
- x being the number of carboxyl functions carried by the poly(carboxylic) acid before the reaction,
- y representing the number of carboxyl functions of the poly(carboxylic) acid which are esterified during the reaction,
- (x-y) being the number of carboxyl functions of the poly(carboxylic) acid which are not esterified or amidifed after the reaction, 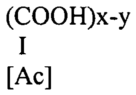
representing the molecular chain of a poly(carboxylic) acid of the formula 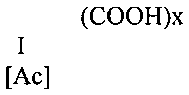 at least two of the carboxyl functions (-COOH) of which have undergone esterification and which carries at least one carboxyl function which has not undergone the esterification reaction;
- [CD] representing the molecular structure of the cyclodextrins, preferably selected from: α-cyclodextrin, β-cyclodextrin, γ-cyclodextrin and mixtures thereof, the derivatives thereof, preferably selected from the aminated, methylated, hydroxypropylated, sulphobutylated, carboxymethylated or carboxylic derivatives thereof, and mixtures thereof, said bioactive agent being a therapeutic molecule which forms a complex with the cyclodextrin molecules and/or cyclodextrin derivatives.

15. Prosthesis (10) according to any one of claims 12 to 14, **characterised in that** the porous material is formed from an assembly, a mesh, a woven material or a knitted material formed from at least one polymer thread, formed in particular on the basis of a polymer selected from the group consisting of polyethylene terephthalate (PET), polyethylene terephthalate glycol (PETG), polyurethane (PU), polylactic acid, polyglycolic acid and the copolymers thereof, polyvinylidene fluoride (PVDF), polytetrafluoroethylene (PTFE), polyethylene glycol (PEG), polyamide 6, polyamide 6,6, polypropylene, polyethylene and the copolymers thereof, or a combination of these polymers.

16. Prosthesis (10) according to any one of claims 12 to 15, **characterised in that** the receiving coating (14) forms between 5 % and 7 % of the total weight of the prosthesis (10).

17. Prosthesis (10) according to any one of claims 12 to 16, **characterised in that** it comprises at least one bioactive agent received in the receiving coating (14).

18. Prosthesis (10) according to any one of claims 12 to 17, **characterised in that** the bioactive agent is selected from anticoagulants, antithrombotics, anti-mitotic agents, anti-proliferation agents, anti-adhesive agents, anti-migration agents, cell adhesion promoters, growth factors, anti-parasitic molecules, anti-inflammatories, anti-fungal agents, anti-microbial molecules, antiseptics and antibiotics.

19. Prosthesis (10) according to any one of claims 12 to 18, **characterised in that** the element (12) is a tubular element, the tubular element (12) provided with said coatings (14, 16) internally delimiting a sealed liquid circulation channel (22).

20. Prosthesis (10) according to any one of claims 12 to 19, **characterised in that** the receiving coating (14) comprises a hydroxypropylated derivative of a β-cyclodextrin.

21. Use of at least one cyclodextrin and/or at least one cyclodextrin derivative and/or at least one cyclodextrin inclusion complex and/or cyclodextrin derivative inclusion complex for producing a bacteriostatic medical prosthesis (10) obtainable by the process according to any one of claims 1 to 11.

22. Use according to claim 21, for producing a bactericidal medical prosthesis (10).

23. Use according to either claim 21 or claim 22, **characterised in that** the medical prosthesis (10) is intended to come into contact with blood plasma or any other biological liquid or tissue.

24. Use according to any one of claims 21 to 23, **characterised in that** the cyclodextrin derivative is a a hydroxypropylated derivative of a β-cyclodextrin.

## Patentansprüche

1. Verfahren zur Herstellung einer implantierbaren Prothese (10) aus einem Grundkörper (12) aus porösem Material, wobei das Verfahren die folgenden aufeinanderfolgenden Schritte umfasst:
a) Aufbringen (50) auf den Körper (12) einer Beschichtung (14) zur Aufnahme einer bioaktiven Substanz, wobei die Aufnahmebeschichtung (14) umfasst:
- wenigstens ein Cyclodextrin und/oder wenigstens ein Cyclodextrin-Derivat und/oder wenigstens einen Einschlusskomplex von Cyclodextrin und/oder von Cyclodextrin-Derivaten,
- wenigstens eine Polycarbonsäure und
- ggf. einen Katalysator;
b) Erhitzen (58) des Körpers (12) auf eine Temperatur zwischen 100 °C und 220 °C über eine Dauer von 1 bis 90 Minuten;
c) Waschen (60) des Körpers (12) mit Wasser;
d) Trocknen (60) des Körpers (12), anschließend
e) ggf. Imprägnieren (46) des Körpers (12) in einer konzentrierten Lösung wenigstens einer bioaktiven Substanz;
**dadurch gekennzeichnet, dass** das Verfahren nach dem Trocknungsschritt einen Schritt (44) des Abscheidens einer zusätzlichen Beschichtung (16) auf dem Körper (12) zur Abdichtung auf der Basis von Kollagen, Gelatine, Polyurethan oder ihren Kombinationen umfasst.

2. Verfahren nach Anspruch 1, **dadurch gekennzeichnet, dass** der Schritt (50) des Aufbringens der Aufnahmebeschichtung (14) das Imprägnieren des Körpers mit einer wässrigen Lösung (52) umfasst, welche umfasst:
- wenigstens ein Cyclodextrin und/oder wenigstens ein Cyclodextrin-Derivat und/oder wenigstens einen Einschlusskomplex von Cyclodextrin und/oder von Cyclodextrin-Derivaten,
- wenigstens eine Polycarbonsäure und
- ggf. einen Katalysator.

3. Verfahren nach einem der Ansprüche 1 bis 2, **dadurch gekennzeichnet, dass** es vor dem Erhitzungsschritt (58) einen Schritt (56) zum Vorbrennen (12) des Körpers bei einer Temperatur zwischen 40 °C und 100 °C umfasst.

4. Verfahren nach einem der vorhergehenden Ansprüche, **dadurch gekennzeichnet, dass** es nach dem Trocknungsschritt (60) einen Schritt (43) des Neutralisierens des Körpers (12) umfasst, um diesen auf einen neutralen pH-Wert zu bringen.

5. Verfahren nach einem der vorhergehenden Ansprüche, **dadurch gekennzeichnet, dass** der Körper (12) von einer Verbindung, einem Geflecht, einem Gewebe oder einem Gestrick aus wenigstens einer Polymerfaser gebildet ist, die insbesondere auf der Basis eines Polymers hergestellt ist, welches aus der Gruppe bestehend aus Polyethylenterephthalat (PET), Polyethylenterephthalat-Glycol (PETG), Polyurethan (PU), Polymilchsäure, Polyglycolsäure und ihren Copolymeren, Polyvinylidenfluorid (PVDF), Polytetrafluorethylen (PTFE), Polyethylenglycol (PEG), Polyamid 6, Polyamid 6,6, Polypropylen, Polyethylen und ihren Copolymeren oder einer Kombination dieser Polymere ausgewählt ist.

6. Verfahren nach einem der vorhergehenden Ansprüche, **dadurch gekennzeichnet, dass** die Aufnahmebeschichtung (14) zwischen 5 % und 7 % der Gesamtmasse der Prothese (10) bildet.

7. Verfahren nach einem der vorhergehenden Ansprüche, **dadurch gekennzeichnet, dass** es den Schritt e) des Imprägnierens (46) des Körpers (12) in einer konzentrierten Lösung wenigstens einer bioaktiven Substanz umfasst.

8. Verfahren nach Anspruch 7, **dadurch gekennzeichnet, dass** die bioaktive Substanz aus den Antikoagulantien, den Antithrombotika, den Mitosehemmern, den Proliferations-, Adhäsions-, Migrationshemmern, den Zelladhäsionspromotoren, den Wachstumsfaktoren, den antiparasitären Molekülen, den Entzündungshemmern, den angiogenen Substanzen, den Angiogenese-Hemmern, den Vitaminen, den Hormonen, den Proteinen, den Fungiziden, den antimikrobiellen Molekülen, den Antiseptika oder den Antibiotika ausgewählt ist.

9. Verfahren nach einem der vorhergehenden Ansprüche, **dadurch gekennzeichnet, dass** es vor dem Schritt (50) des Aufbringens der Aufnahmebeschichtung (14) und/oder nach dem Waschschritt einen Schritt (40) zum Prägen des Körpers (12) umfasst.

10. Verfahren nach einem der vorhergehenden Ansprüche, **dadurch gekennzeichnet, dass** der Körper (12) ein röhrenförmiger Körper ist, wobei der mit den Beschichtungen (14, 16) versehene röhrenförmige Körper (12) innen eine dichte Leitung (22) für das Zirkulieren einer Flüssigkeit begrenzt.

11. Verfahren nach einem der vorhergehenden Ansprüche, **dadurch gekennzeichnet, dass** die Aufnahmebeschichtung (14) ein Hydroxypropyl-Derivat von β-Cyclodextrin umfasst.

12. Implantierbare Prothese (10), umfassend:
- einen Grundkörper (12) aus porösem Material,
- eine auf den Körper (12) aufgebrachte Beschichtung (14) zur Aufnahme einer bioaktiven Substanz, wobei die Beschichtung wenigstens ein Cyclodextrin und/oder wenigstens ein Cyclodextrin-Derivat und/oder wenigstens einen Einschlusskomplex von Cyclodextrin und/oder von Cyclodextrin-Derivaten umfasst,
- ggf. wenigstens eine bioaktive Substanz, die in der Aufnahmebeschichtung (14) aufgenommen ist;
**dadurch gekennzeichnet, dass** die Prothese (10) eine auf dem Körper (12) abgeschiedene zusätzliche Abdichtungsbeschichtung (16) auf der Basis von Kollagen, Gelatine, Polyurethan oder ihren Kombinationen umfasst.

13. Prothese (10) nach Anspruch 12, **dadurch gekennzeichnet, dass** der Körper (12) eine chemische Struktur aufweist, die eine Hydroxylfunktion und/oder eine Aminofunktion umfasst, wobei die Struktur an wenigstens ein Cyclodextrinmolekül oder an ein aus Cyclodextrin zusammengesetztes Polymer der Aufnahmebeschichtung kovalent gebunden ist, dessen Struktur die Wiederholung des Musters der allgemeinen Formel 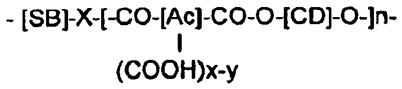 aufweist, wobei SB die chemische Struktur des aus einem Polymermaterial natürlichen oder künstlichen Ursprungs gebildeten Körpers darstellt, wobei X entweder ein Sauerstoffatom oder eine NH-Gruppe, x > 3 und 2 < y < (x-1) und (x-y) > 1 ist,
- wobei x die Anzahl der von der Polycarbonsäure getragenen Carboxylfunktionen vor der Reaktion ist,
- y die Anzahl der während der Reaktion veresterten oder amidierten Carboxylfunktionen der Polycarbonsäure darstellt,
- (x-y) die Anzahl der nach der Reaktion nicht veresterten oder amidierten Carboxylfunktionen der Polycarbonsäure ist,
wobei 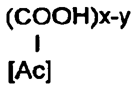 die Molekülkette einer Polycarbonsäure der Formel 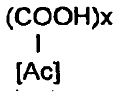 darstellt, von der wenigstens zwei Carboxylfunktionen (-COOH) eine Veresterung erfahren haben oder eine Veresterung bzw. eine Amidierung erfahren haben, und die wenigstens eine Carboxylfunktion trägt, die keine Veresterungs- oder Amidierungsreaktion erfahren hat;
- wobei [CD] die Molekülstruktur der Cyclodextrine darstellt, die vorzugsweise ausgewählt sind aus: α-Cyclodextrin, β-Cyclodextrin, γ-Cyclodextrin und ihren Mischungen; ihren Derivaten, die vorzugsweise aus den Amino-, Methyl-, Hydroxypropyl-, Sulfobutyl-, Carboxymethyl- oder Carboxylderivaten und ihren Mischungen ausgewählt sind, wobei die bioaktive Substanz wenigstens ein therapeutisches Molekül ist, das mit den Cyclodextrin- und/oder Cyclodextrin-Derivat-Molekülen einen Komplex bildet.

14. Prothese (10) nach einem der Ansprüche 12 oder 13, **dadurch gekennzeichnet, dass** die Aufnahmebeschichtung (14) von einem vernetzten Polymer von wenigstens einem Cyclodextrin und/oder wenigstens einem Cyclodextrin-Derivat und/oder wenigstens einem Einschlusskomplex von Cyclodextrin oder von Cyclodextrin-Derivaten gebildet ist, dessen Struktur die Wiederholung eines Musters der allgemeinen Formel 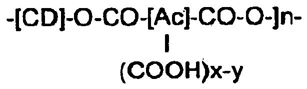 umfasst, mit x > 3 und 2 < y ≤ (x-1) und (x-y) ≥ 1,
- wobei x die Anzahl der von der Polycarbonsäure getragenen Carboxylfunktionen vor der Reaktion ist,
- wobei y die Anzahl der während der Reaktion veresterten Carboxylfunktionen der Polycarbonsäure darstellt,
- wobei (x-y) die Anzahl der nach der Reaktion nicht veresterten Carboxylfunktionen der Polycarbonsäure ist,
wobei 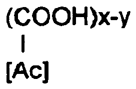 die Molekülkette einer Polycarbonsäure der Formel 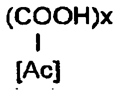 darstellt, von der wenigstens zwei Carboxylfunktionen (-COOH) eine Veresterung erfahren haben und die wenigstens eine Carboxylfunktion trägt, die keine Veresterungsreaktion erfahren hat;
- wobei [CD] die Molekülstruktur der Cyclodextrine darstellt, die vorzugsweise ausgewählt sind aus: α-Cyclodextrin, β-Cyclodextrin, γ-Cyclodextrin und ihren Mischungen; ihren Derivaten, die vorzugsweise aus den Amino-, Methyl-, Hydroxypropyl-, Sulfobutyl-, Carboxymethyl- oder Carboxylderivaten und ihren Mischungen ausgewählt sind, wobei die bioaktive Substanz ein therapeutisches Molekül ist, das mit den Cyclodextrin- und/oder Cyclodextrin-Derivat-Molekülen einen Komplex bildet.

15. Prothese (10) nach einem der Ansprüche 12 bis 14, **dadurch gekennzeichnet, dass** das poröse Material von einer Verbindung, einem Geflecht, einem Gewebe oder einem Gestrick aus wenigstens einer Polymerfaser gebildet ist, die insbesondere auf der Basis eines Polymers hergestellt ist, welches aus der Gruppe bestehend aus Polyethylenterephthalat (PET), Polyethylenterephthalat-Glycol (PETG), Polyurethan (PU), Polymilchsäure, Polyglycolsäure und ihren Copolymeren, Polyvinylidenfluorid (PVDF), Polytetrafluorethylen (PTFE), Polyethylenglycol (PEG), Polyamid 6, Polyamid 6,6, Polypropylen, Polyethylen und ihren Copolymeren oder einer Kombination dieser Polymere ausgewählt ist.

16. Prothese (10) nach einem der Ansprüche 12 bis 15, **dadurch gekennzeichnet, dass** die Aufnahmebeschichtung (14) zwischen 5 % und 7 % der Gesamtmasse der Prothese (10) bildet.

17. Prothese (10) nach einem der Ansprüche 12 bis 16, **dadurch gekennzeichnet, dass** sie wenigstens eine in der Aufnahmebeschichtung (14) aufgenommene bioaktive Substanz umfasst.

18. Prothese (10) nach einem der Ansprüche 12 bis 17, **dadurch gekennzeichnet, dass** die bioaktive Substanz aus den Antikoagulantien, den Antithrombotika, den Mitosehemmern, den Proliferations-, Adhäsions-, Migrationshemmern, den Zelladhäsionspromotoren, den Wachstumsfaktoren, den antiparasitären Molekülen, den Entzündungshemmern, den Fungiziden, den antimikrobiellen Molekülen, den Antiseptika und den Antibiotika ausgewählt ist.

19. Prothese (10) nach einem der Ansprüche 12 bis 18, **dadurch gekennzeichnet, dass** der Körper (12) ein röhrenförmiger Körper ist, wobei der mit den Beschichtungen (14, 16) versehene röhrenförmige Körper (12) innen eine dichte Leitung (22) für das Zirkulieren einer Flüssigkeit begrenzt.

20. Prothese (10) nach einem der Ansprüche 12 bis 19, **dadurch gekennzeichnet, dass** die Aufnahmebeschichtung (14) ein Hydroxypropyl-Derivat von β-Cyclodextrin umfasst.

21. Verwendung wenigstens eines Cyclodextrins und/oder wenigstens eines Cyclodextrin-Derivats und/oder wenigstens eines Einschlusskomplexes von Cyclodextrin und/oder von Cyclodextrin-Derivaten für die Herstellung einer implantierbaren bakteriostatischen Prothese (10), die geeignet ist, mittels eines Verfahrens nach einem der Ansprüche 1 bis 11 erhalten zu werden.

22. Verwendung nach Anspruch 21, für die Herstellung einer implantierbaren bakteriziden Prothese (10).

23. Verwendung nach einem der Ansprüche 21 oder 22, **dadurch gekennzeichnet, dass** die implantierbare Prothese (10) dazu bestimmt ist, mit Blutplasma oder jedweden anderen Flüssigkeit oder biologischem Gewebe in Kontakt zu kommen.

24. Verwendung nach einem der Ansprüche 21 bis 23, **dadurch gekennzeichnet, dass** das Cyclodextrin-Derivat ein Hydroxypropylderivat von β-Cyclodextrin ist.
